# EUROPEAN PATENT APPLICATION

(11) **EP 4 729 624 A1**
(43) Date of publication of application: **22.04.2026**
(21) Application number: 24832473.3
(22) Date of filing: 27.06.2024
(51) Int. Cl.: C12P 13/08, C07C 227/42, C07C 229/08

(54) **METHOD FOR PREPARING L-VALINE CRYSTALS AND/OR GRANULES USING PH ADJUSTMENT AND PVA ADDITION**

(30) Priority: 28.06.2023 KR 20230083411
(71) Applicant: CJ Cheiljedang Corporation, Seoul 04560 (KR)
(72) Inventor: JEONG, Jae Yun, Seoul 04560 (KR); KANG, Seung Hoon, Seoul 04560 (KR); KIM, Sumin, Seoul 04560 (KR); KANG, Ji-hun, Seoul 04560 (KR)
(74) Representative: Meissner Bolte Partnerschaft mbB
(86) International application number: PCT/KR2024/009022
(87) International publication number: WO 2025/005696

(57) **Abstract**

The present disclosure relates to a method for preparing valine crystals and/or valine granules using pH adjustment and PVA addition.

## Description

### [Technical Field]

The present disclosure relates to a method for preparing valine crystals and granules that is capable of reducing the amount of steam required during valine granule production and increasing the efficiency of valine granule production by forming valine crystals and mixed granules with low moisture contents.

### [Background Art]

To obtain high-purity amino acids through fermentation, it is essential to carry out a separation or purification step of the amino acids for removing by-products after the fermentation process.

However, for feed-grade amino acid products, a product containing a high amino acid content (98% or more) is not required, and it may be more appropriate to produce a granulated product having a high content (70% or more) that comprises other valuable nutrients in the fermentation broth without generating waste products in the purification step.

In the conventional technology (US 7,514,111 B2), in order to produce a granulated amino acid product for use as a feed additive, a large amount of moisture in the fermentation broth is evaporated, and granules are then produced using a fluidized bed granulator. In the case of highly soluble amino acids, crystals do not form even when moisture is evaporated until the solid content reaches about 60% to 65% (moisture content 35% to 40%), and therefore granulation can be achieved by a fluidized bed granulation method in which the fermentation broth is sprayed through a nozzle. However, since valine has low solubility, crystals form even when the solid content in the fermentation broth is as low as about 12% to 15% (moisture content 85% to 88%), and therefore the moisture content of the fermentation broth cannot be reduced in order to prevent crystal formation. Accordingly, a large amount of water must be evaporated during the drying process, and thus a significant amount of energy is required for drying.

### [Prior Art Documents]

### [Patent Documents]

(Patent Document 1) US 7,514,111 B2
(Patent Document 2) US 10,072,278 B2

### [Disclosure]

### [Technical Problem]

One object of the present disclosure is to provide a method for preparing valine crystals, comprising: a step of preparing a fermentation broth comprising valine; a step of adjusting the pH of the fermentation broth; a step of adding polyvinyl alcohol (PVA) to the fermentation broth; and a step of separating valine crystals comprising moisture from the fermentation broth.

Another object of the present disclosure is to provide a method for preparing valine granules, comprising: a step of preparing a fermentation broth comprising valine; a step of adjusting the pH of the fermentation broth; a step of adding polyvinyl alcohol (PVA) to the fermentation broth; a step of separating valine crystals comprising moisture from the fermentation broth; a step of preparing mixed granules by mixing the valine crystals with a seed; and a step of drying the valine mixed granules.

### [Technical Solution]

The present disclosure provides a method for preparing valine crystals, comprising: a step of preparing a fermentation broth comprising valine; a step of adjusting the pH of the fermentation broth; a step of adding polyvinyl alcohol (PVA) to the fermentation broth; and a step of separating valine crystals comprising moisture from the fermentation broth.

The present disclosure provides a method for preparing valine granules, comprising: a step of preparing a fermentation broth comprising valine; a step of adjusting the pH of the fermentation broth; a step of adding polyvinyl alcohol (PVA) to the fermentation broth; a step of separating valine crystals comprising moisture from the fermentation broth; a step of preparing mixed granules by mixing the valine crystals with a seed; and a step of drying the valine mixed granules.

### [Advantageous Effects]

According to the present disclosure, by adjusting the pH of the fermentation broth comprising valine and adding PVA, the moisture content of valine crystals or valine mixed granules can be reduced, thereby reducing the energy required for drying.

### [Brief Description of the Drawings]

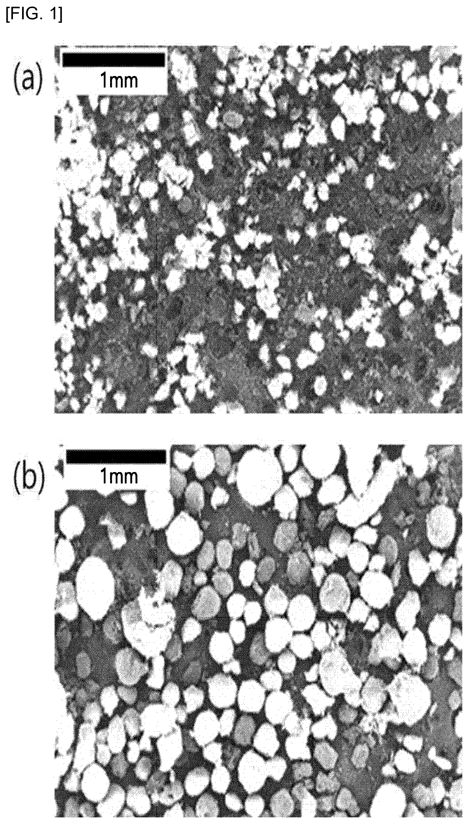

FIG. 1 shows SEM images of valine crystals prepared according to an embodiment of the present disclosure. FIG. 1(a) and FIG. 1(b) each represent valine crystals prepared by no addition or 2 wt% addition of PVA to the culture broth.

### [Detailed Description of Preferred Embodiments]

The present disclosure will be described in detail as follows. Meanwhile, each description and embodiment described herein can be applied to other descriptions and embodiments, respectively. That is, all combinations of various elements described herein fall within the scope of the present disclosure. Further, the scope of the present disclosure is not limited by the specific description described below.

Additionally, a number of papers and patent documents have been cited throughout the present specification. The content of the cited papers and patent documents is incorporated herein by reference in its entirety to describe the level of the technical field to which the present disclosure belongs and the contents of the present disclosure more clearly.

Furthermore, those skilled in the art may be able to recognize or identify numerous equivalents to the particular embodiments of the present disclosure described herein by using routine experimentation. Moreover, these equivalents are intended to be included in the present disclosure.

As used in the specification and appended claims of the present disclosure, the singular articles ("a," "an," and "the") include plural referents unless the context clearly indicates otherwise. Further, unless the context indicates otherwise, singular terms include their plural forms, and plural terms include their singular forms. As used in the specification and appended claims of the present disclosure, the use of "or" may include the meaning of "and/or", unless indicated otherwise.

As used herein, the term "about" may precede a specific numerical value. As used herein, the term "about" encompasses not only the precise numerical value that is specified after the term but also a range that is approximately or nearly close to that value. Considering the context in which the number is presented, it can be determined whether the specific number mentioned is close to or nearly that number. For example, the term "about" can refer to a range of -10% to +10% of the numerical value. As another example, the term "about" may refer to a range of -5% to +5% of the given numerical value, but is not limited thereto.

The method for preparing valine crystals according to the present disclosure comprises a step of preparing a fermentation broth comprising valine; a step of adjusting the pH of the fermentation broth; a step of adding polyvinyl alcohol (PVA) to the fermentation broth; and a step of separating valine crystals comprising moisture from the fermentation broth. Hereinafter, each step is described in detail.

The step of preparing a fermentation broth is a step of preparing a fermentation broth comprising valine. The valine comprised in the fermentation broth is an amino acid with low solubility in water. In particular, low solubility does not mean that the solubility is equal to or less than a specific standard. The fermentation broth prepared in the above step may be a fermentation product prepared by the metabolism of a microbial strain. As used herein, the term "fermentation product" may refer to a product formed by enzymatic or metabolic decomposition of organic substances using a microorganism, for example, a microorganism producing valine. For example, a fermentation product may comprise a culture itself obtained by culturing microorganisms in a culture medium, or a culture obtained after removing the microbial strain therefrom, a concentrate, dried product, or freeze-dried product thereof. Further, in this case, the fermentation broth may comprise the entire fermentation product comprising valine, or may be a fermentation product comprising valine from which impurities have been removed.

The fermentation broth comprises valine as a main component but may additionally comprise other components. For example, in the fermentation broth, other amino acids, organic acids, and inorganic substances may be further provided in addition to valine, and these substances may be in a state dissolved in water. The concentration of valine in the fermentation broth may be about 1 g/L to about 200 g/L. However, the above-described concentration of valine is merely illustrative, and a fermentation broth having a concentration outside the above range may also be used in the method for producing valine crystals according to an embodiment of the present disclosure.

The "microorganism producing valine" or "microorganism having valine-producing ability" used in the step for preparing a fermentation broth of the present disclosure comprises both wild-type microorganisms naturally having valine-producing ability and microorganisms that have undergone natural or artificial genetic modification and in which valine-producing ability was imparted to a parent strain without valine-producing ability. It may be a microorganism comprising genetic modification for the production of an amino acid, i.e., valine, in which a specific mechanism is weakened or enhanced due to factors such as the insertion of an exogenous gene or the enhancement or inactivation of endogenous gene activity. Specifically, in the present disclosure, the microorganism producing valine or the microorganism with valine-producing ability may be a microorganism in which some genes in the biosynthetic pathway for valine are enhanced or attenuated or some genes in the degradation pathway for valine are enhanced or attenuated. "Enhancing" or "increasing" the valine-producing ability of the microorganism of the present disclosure means that the valine-producing ability of the microorganism of the present disclosure is improved compared to that of a microorganism, parent strain, or non-modified microorganism other than the microorganism of the present disclosure. For example, the microorganism of the present disclosure may have a valine-producing ability that is improved by about 1% or more, 10% or more, 100% or more, 200% or more, 500% or more, 1000% or more, 1100% or more, 1200% or more, or 1300% or more, compared to the valine-producing ability of another microorganism, and may have a valine-producing ability that is improved by about 1.01 times or more, 2 times or more, 5 times or more, 10 times or more, 11 times or more, 12 times or more, or 13 times or more, but is not limited thereto. The term "about" refers to a range encompassing ±0.5, ±0.4, ±0.3, ±0.2, ±0.1, *etc.,* including all numerical values in a range equal to or similar to the numerical value following the term "about", but the range is not limited thereto.

The above-described microorganism used in the step of preparing a fermentation broth of the present disclosure may be at least one selected from the group consisting of the yeast *Candida famata,* the ascomycetes *Eremothecium ashbyii* and *Ashbya gossypii,* and the bacterium *Bacillus subtilis,* and a microorganism of the genus *Corynebacterium.*

For example, when the microorganism used in the step of preparing a fermentation broth of the present disclosure is a microorganism of the genus *Corynebacterium,* the microorganism may specifically be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris, Corynebacterium crenatum,* or *Corynebacterium flavescens,* or more specifically *Corynebacterium glutamicum,* but is not limited thereto.

The microorganism of the genus *Corynebacterium,* particularly *Corynebacterium glutamicum,* is a Gram-positive microorganism widely used for the production of L-amino acids and other useful substances. For the production of the L-amino acids and other useful substances, various studies have been conducted to develop microorganisms with high production efficiency and fermentation process technologies. For example, substance-specific approaches have mainly been employed, such as increasing the expression of genes encoding enzymes involved in the biosynthesis of L-valine or eliminating genes that are unnecessary for the biosynthesis. In the present disclosure, a fermentation broth comprising an amino acid can be prepared using a strain of the genus *Corynebacterium.*

According to an embodiment of the present disclosure, *Corynebacterium glutamicum* used in the preparation of a fermentation broth comprising valine may be a strain for L-valine biosynthesis overexpression in which the regulatory region of L-valine biosynthesis is inactivated by transforming *Corynebacterium glutamicum* ATCC13032, but is not limited thereto. Alternatively, the *Corynebacterium glutamicum* may be a strain transformed with pDZDvalL, pDZDvalP, or pDZDvalS vector using, as a template, the chromosome of *Corynebacterium glutamicum* KCCM11201P, *Corynebacterium glutamicum* KCCM11336P, *Corynebacterium glutamicum* KCCM11337P, *Corynebacterium glutamicum* KCCM11338P, or *Corynebacterium glutamicum* KCCM11201P_DvalA. In another example, the *Corynebacterium glutamicum* may be *Corynebacterium glutamicum* ATCC13032_DvaIL, *Corynebacterium glutamicum* ATCC13032_DvaIP, or *Corynebacterium glutamicum* ATCC13032_DvalS, but is not limited thereto, and comprises, without limitation, a microorganism that is capable of producing a fermentation broth comprising valine (US 10,072,278 B2).

The step of preparing a fermentation broth may further comprise culturing a "microorganism producing valine". The culturing of the microorganism may be performed in a suitable medium known in the art under suitable culturing conditions known in the art. Such a culturing process may be readily adjusted and used by those skilled in the art according to the selected strain. Specifically, the culturing may be batch culturing, continuous culturing, or fed-batch culturing, but is not limited thereto.

As used herein, the term "medium" refers to a mixed substance containing nutrients required to culture the microorganism as a main component, and the medium supplies nutrients, growth factors, *etc*., including water, which are indispensable for survival and development. Specifically, any medium and culture conditions may be used for culturing the microorganism of the present disclosure without particular limitation, as long as the medium is used for the common culture of microorganisms. The microorganism of the present disclosure may be cultured in a common medium containing suitable carbon sources, nitrogen sources, phosphorus sources, inorganic compounds, amino acids, and/or vitamins, *etc.,* while controlling the temperature, pH, *etc.* under aerobic conditions.

As used herein, the term "fermentation broth comprising valine" may be used interchangeably with "valine-containing fermentation broth" or "valine fermentation broth".

Subsequently, a step of adjusting the pH of the fermentation broth is performed. Herein, the pH of the fermentation broth may be adjusted to a pH greater than about 3.0 and less than 6.0. In some cases, the pH of the fermentation broth may be adjusted to about 3.5 to less than 6.0, about 4.0 to less than 6.0, about 4.5 to less than 6.0, about 5.0 to less than 6.0, about 5.5 to less than 6.0, greater than about 3.0 to about 5.5, about 3.5 to about 5.5, about 4.0 to about 5.5, about 4.5 to about 5.5, about 5.0 to about 5.5, about 3.0 to about 5.0, about 3.5 to about 5.0, about 4.0 to about 5.0, about 4.5 to about 5.0, greater than about 3.0 to about 4.5, about 3.5 to about 4.5, about 4.0 to about 4.5, greater than about 3.0 to about 4.0, about 3.5 to about 4.0, or greater than about 3.0 to about 3.5.

By adjusting the pH of the fermentation broth, the size and hardness of the valine crystals formed in the subsequent concentrating step can be adjusted. Specifically, large and hard valine crystals may be formed when performing concentration after adjusting the pH of the fermentation broth to the above-described range, and the moisture content of the separated valine crystals can thereby be reduced. When the pH of the fermentation broth lies outside the above-described range, the crystal size of the valine crystals precipitated after the concentration is small, and the separation of valine crystals from the mother liquor of the fermentation broth may thus be difficult. In addition, since a relatively large amount of moisture is separated with the valine crystals, a lot of energy is required for subsequent drying.

In the step of adjusting the pH of the fermentation broth, pH adjustment may be performed using a conventional acidic substance or basic substance. Meanwhile, the substance added to the fermentation broth for pH adjustment may be selected insofar as it does not affect the physical properties or chemical structure of valine comprised in the fermentation broth. In addition, considering that a valine product may be used for food or feed, an edible acidic substance or basic substance may be added.

After the above-described pH adjustment, processes including heating the fermentation broth may be performed, if necessary, to completely dissolve valine that may have precipitated in the fermentation broth. For example, the fermentation broth may be heated to a temperature of about 60°C to about 90°C.

After performing the step of adjusting the pH of the fermentation broth, a step of adding polyvinyl alcohol (PVA) to the prepared fermentation broth is performed. For example, in the method of the present disclosure, the addition of PVA is intended to adjust the crystallinity of the L-valine crystals to be formed, wherein the addition of PVA allows the formation of L-valine crystals that exhibit uniform and excellent crystal properties without being affected by the concentration rate during the concentration.

In particular, the PVA may be added in an amount of 0.1 wt% to 5 wt% based on the total weight of the fermentation broth. In some cases, the PVA may be added in an amount of 0.3 wt% to 4 wt%, 0.5 wt% to 3 wt%, 0.5 wt% to 2.5 wt%, 1 wt% to 3 wt%, 1 wt% to 2.8 wt%, 1.5 wt% to 3 wt%, 1.5 wt% to 2.5 wt%, 1 wt% to 2.5 wt%, or 1.8 wt% to 2.3 wt%.

For example, L-valine crystals prepared by adding PVA into a culture broth and concentrating according to the method of the present disclosure may be crystals in which the moisture content of the crystals is reduced by 8% to 40% compared with the crystals prepared according to a method that does not involve adding PVA in the second step.

For example, the method of the present disclosure may further comprise a step of concentrating after the step of adding PVA to the fermentation broth. The concentrating step is intended to form L-valine crystals, and the concentration may be performed at a solid content of 15% to 30%, but is not limited thereto.

The concentrating step removes moisture in the fermentation broth, precipitating valine in the form of crystals. The concentration may be performed by various methods. The concentration may be performed, at the discretion of those skilled in the art, in a conventional concentrator (for example, a paddle dryer, a slurry drying apparatus, a vacuum concentrator, a forced-circulation concentrator, a thin-film concentrator, or a rotary concentrator).

In the concentrating step, the concentration may be performed by removing moisture in the fermentation broth at a rate of 30 g/L/hr to 150 g/L/hr (meaning that 30 g to 150 g of moisture per liter of the fermentation broth is removed over 1 hour). In some cases, the concentration may be performed by removing moisture in the fermentation broth at a rate of 90 g/L/hr to 150 g/L/hr, 120 g/L/hr to 150 g/L/hr, 60 g/L/hr to 120 g/L/hr, 90 g/L/hr to 120 g/L/hr, or 60 g/L/hr to 90 g/L/hr. In the present disclosure, the rate of concentration is a factor that affects the size of valine crystals. When concentrated at the above-described rate, valine crystals relatively large in size are formed, and can be easily separated from the mother liquor of the fermentation broth. In addition, the moisture in the valine crystals decreases accordingly.

Subsequently, a step of separating valine crystals comprising moisture from the fermentation broth is performed. The valine crystals may be valine crystals in which moisture in the crystals is reduced by concentration.

In the separating step, valine crystals comprising moisture are separated from the fermentation broth, and in particular, a solid-liquid separator such as a vacuum membrane filtration apparatus, a pressure membrane filtration apparatus, or a centrifugation apparatus may be used. However, the above-described methods are merely illustrative, and the method of performing the separating step is not limited to the above-described examples.

In the separating step, the mother liquor remaining after separating the valine crystals may be reused in the concentration. Thus, valine that has failed to form crystals comprising moisture in the concentrating step or valine that has precipitated as crystals with a size below a specific value, remaining in the mother liquor without being separated in the step of separating valine crystals, may be recycled. In addition, after circulation of the mother liquor, a process such as heating the fermentation broth may additionally be performed, if necessary, to re-dissolve the valine precipitated in the form of fine crystals.

According to the method for preparing valine crystals according to an embodiment of the present disclosure, valine crystals that have low moisture content and are easily separable can be obtained by adjusting the pH of the fermentation broth comprising valine and adding an appropriate amount of PVA to the fermentation broth.

The valine crystals produced according to an embodiment of the present disclosure may have a viscosity of greater than 0 cP and less than 80 cP. In some cases, the viscosity of the valine crystals may be greater than 20 cP to less than 80 cP, greater than 40 cP to less than 80 cP, greater than 60 cP to less than 80 cP, greater than 0 cP to 70 cP, greater than 20 cP to 70 cP, greater than 40 cP to 70 cP, greater than 60 cP to 70 cP, greater than 0 cP to 50 cP, greater than 20 cP to 50 cP, greater than 40 cP to 50 cP, greater than 0 cP to 30 cP, greater than 20 cP to 30 cP, or greater than 0 cP to 10 cP. The valine crystals having a viscosity within the above-described range may be easily separated from the mother liquor due to their low moisture content and appropriate size.

The valine crystals prepared according to an embodiment of the present disclosure may comprise 10 wt% to 55 wt% of moisture. In some cases, the valine crystals may comprise moisture in an amount of 20 wt% to 55 wt%, 30 wt% to 55 wt%, 40 wt% to 55 wt%, 50 wt% to 55 wt%, 10 wt% to 50 wt%, 20 wt% to 50 wt%, 30 wt% to 50 wt%, 40 wt% to 50 wt%, 10 wt% to 40 wt%, 20 wt% to 40 wt%, 30 wt% to 40 wt%, 10 wt% to 30 wt%, 20 wt% to 30 wt%, or 10 wt% to 20 wt%. The valine crystals having moisture in the above-described amount have a relatively low moisture content, and may be used directly without a separate drying step or may be used after minimal drying, thereby reducing the energy required for the process.

Further, the method of the present disclosure may additionally comprise a drying step, a sieving step, or both after the step of separating the L-valine crystals. Each step can be performed using methods known in the art without limitation (for example, US 2021-0094903 A1). The specific conditions may be appropriately changed to optimize the process, but are not limited thereto.

The present disclosure provides L-valine crystals comprising PVA and L-valine and having a moisture content that is reduced by 5% to 40% compared with L-valine crystals prepared without using PVA during concentration.

For example, the L-valine crystals may have a spherical particle form.

Specifically, the L-valine crystals of the present disclosure may be prepared by the above-described method. Further, the L-valine crystals of the present disclosure may be used for feed or as a feed additive, but the use is not limited thereto.

The method for preparing valine granules comprises: a step of preparing a fermentation broth comprising valine; a step of adjusting the pH of the fermentation broth; a step of adding polyvinyl alcohol (PVA) to the fermentation broth; a step of separating valine crystals comprising moisture from the fermentation broth; a step of preparing mixed granules by mixing the valine crystals with a seed; and a step of drying the valine mixed granules.

In particular, the step of preparing a fermentation broth, the step of adjusting the pH of the fermentation broth, the step of adding PVA to the fermentation broth, and the step of separating valine crystals may be carried out in the same manner as in the method for producing valine crystals described above. Accordingly, to avoid redundancy, the following description focuses on the step of preparing mixed granules by mixing the valine crystals with a seed and the step of drying the mixed granules.

In the step of preparing mixed granules, the valine crystals obtained in the step of separating valine crystals are mixed with a seed to prepare valine granules.

The seed used in the step of preparing mixed granules, which is also referred to as a seed crystal or nucleus crystal, may refer to a substance used as a catalyst for crystallization or granulation of a liquid. In the present disclosure, the seed may be valine crystals. When brought into contact with the fermentation broth, the seed can form granules as solid components present in the fermentation broth bind to the seed and thereby undergo agglomeration.

The seed used in the step of preparing mixed granules may have an average particle size of 150 µm to 300 µm. Specifically, seeds with an average particle size of 150 µm to 250 µm, 200 µm to 300 µm, or 200 µm to 250 µm may be used, but are not limited thereto. The particle size of the seed used may affect the productivity of granule production according to the present disclosure, and therefore may be appropriately selected by those skilled in the art in consideration of desired moisture content and the like.

In the step of preparing mixed granules, a mixer granulator may be used. The mixer granulator may obtain granules by supplying the valine crystals obtained in the preceding separating step while simultaneously feeding the seed into the mixer granulator through a feeder at a constant rate. The mixer granulator may comprise a feeder for supplying the seed into the interior and a member for supplying valine crystals. In addition, in the interior of the mixer granulator, it may be provided with a gas nozzle for generating an air flow inside the chamber, paddles for mixing the seed and the valine crystals provided in the chamber, and the like. The valine mixed granules may be formed by the agglomeration of the seed with the valine crystals fed into the chamber.

According to an embodiment of the present disclosure, valine mixed granules with low moisture content and large crystal size may be formed by the mixing of the seed and valine crystals comprising moisture. As reviewed above, valine crystals having large crystal size can be precipitated according to the present disclosure, and valine crystals with large crystal size can be easily separated from the fermentation broth; accordingly, the moisture content in the valine crystals is relatively low. In addition, in the step of preparing mixed granules, the valine crystals with low moisture content are granulated with the seed in the mixer granulator, and the resulting valine mixed granules also have low moisture content and a large size.

In particular, the "granules" refer to macroscopic particles that are large permanent agglomerates, formed by the aggregation of small particles such as powders, and may be particles having an average particle diameter of 50 µm to 5 mm, 75 µm to 4 mm, or 100 µm to 3 mm.

The valine mixed granules obtained in the step of preparing mixed granules may comprise moisture in an amount of 10 wt% to 50 wt%. In some cases, the valine mixed granules may comprise moisture in an amount of 20 wt% to 50 wt%, 30 wt% to 50 wt%, 40 wt% to 50 wt%, 10 wt% to 40 wt%, 20 wt% to 40 wt%, 30 wt% to 40 wt%, 10 wt% to 30 wt%, 20 wt% to 30 wt%, 10 wt% to 40 wt%, 20 wt% to 40 wt%, 30 wt% to 40 wt%, 10 wt% to 30 wt%, 20 wt% to 30 wt%, or 10 wt% to 20 wt%. These moisture contents are low compared to that of the valine granules obtained according to conventional technology. Accordingly, the amount of energy required to dry the valine mixed granules obtained according to the present disclosure can be reduced.

Subsequently, a drying step of drying the valine mixed granules is performed.

In the drying step, the valine mixed granules obtained above are dried. The drying method is not limited.

One aspect of the present disclosure may provide a valine product comprising valine mixed granules. As used herein, the term "valine product" refers to a product in which the valine comprised in the fermentation broth has been formulated into various dosage forms. For example, the valine product may refer to a valine-containing mixture in the form of granules. However, if necessary, the dosage form of the valine product may be varied insofar as it does not alter the spirit of the invention of the present disclosure. In addition, as described above, further subsequent processes may be performed to implement various forms of the valine product. The above-described valine product may be used as an animal feed additive, *etc.,* and the use thereof is not limited.

According to the present disclosure, valine mixed granules with large size and low moisture content can be prepared by concentrating the fermentation broth comprising valine, separating valine crystals, and mixing the separated valine crystals with a seed. The valine mixed granules with low moisture content have the advantage of requiring a reduced amount of energy for drying. In one example, according to the present disclosure, the moisture content of the valine mixed granules can be further reduced by adjusting the pH of the fermentation broth or controlling the concentration rate.

The L-valine crystals or granules of the present disclosure may be suitable for use as a feed additive in animal feed preparation. For example, as the feed additive, the L-valine crystals may be mixed with feed materials by themselves as part of an animal feed premix or as a precursor of animal feed. The feed composition comprising the L-valine crystals or granules may be administered to an animal either alone or in combination with other feed additives in edible carriers. Additionally, the feed composition may be readily administered to an animal as top dressing, by directly mixing it into the animal feed, or in a separate oral formulation.

### [Mode for Carrying Out the Invention]

Hereinafter, the present disclosure is explained in more detail by the following examples. However, these examples are set forth to illustrate the present disclosure, and the scope of the present disclosure is not limited thereto. Meanwhile, each description and embodiment described herein can be applied to other descriptions and embodiments, respectively. That is, all combinations of various elements described herein fall within the scope of the present disclosure. Additionally, a number of papers and patent documents have been cited throughout the present specification. The content of the cited papers and patent documents is incorporated herein by reference in its entirety to describe the level of the technical field to which the present disclosure belongs and the contents of the present disclosure more clearly.

### Experimental Example 1: Preparation of valine fermentation broth through culturing a microorganism of the genus Corynebacterium

In this Experimental Example, in order to prepare granules comprising L-valine, *Corynebacterium glutamicum* KCCM11338P (US 10072278 B2), an L-valine-producing strain, was cultivated at about 30°C for 72 hours in a medium composed of 5% glucose, 2% ammonium sulfate, 0.1% potassium dihydrogen phosphate, 0.05% magnesium sulfate heptahydrate, 2.0% corn steep liquor (CSL), and 200 µg/L biotin, with a pH of 7.2, thereby obtaining a fermentation broth having the following composition. The fermentation broth comprised the culture medium and the microorganism, and the moisture content and composition thereof were analyzed. Table 1 shows the results of the composition analysis of the valine fermentation broth.

**[Table 1]**

| Composition | Content (g/L) |
|---|---|
| Valine | 80.0 |
| Other amino acids | 8.8 |
| Organic acids | 0.7 |
| Minerals | 11.0 |
| Moisture (%) | 89.0 |

### Experimental Example 2: Comparison of moisture content in valine crystals according to the pH of the fermentation broth

The fermentation broth prepared in Experimental Example 1 with an L-valine concentration of 80 g/L was divided into 2 L portions, and sulfuric acid was added thereto to prepare process solutions with different pHs (7.5 to 3.0). To precipitate valine crystals in the prepared process solutions, the solutions were concentrated using a vacuum concentrator at a vacuum degree of 120 torr and at a rate of 60 g/L/hr until the valine concentration of the process solution reached 200 g/L. The concentrated crystal slurry comprising valine crystals was subjected to solid-liquid separation using a basket centrifuge to obtain the L-valine crystals and the mother liquor.

The moisture content in the valine crystals according to the pH of the fermentation broth was compared through LOD analysis (Table 2).

**[Table 2]**

| | | | | | |
|---|---|---|---|---|---|
| pH of fermentation broth | 7.0 | 6.0 | 5.0 | 4.0 | 3.0 |
| Slurry viscosity (cP) | 89 | 80 | 20.5 | 16.0 | 90 |
| Moisture content in crystals (wt%) | 45 | 40 | 32 | 30 | 39 |

The results of the LOD moisture measurement confirmed that the moisture content in the valine crystals was the lowest when the pH of the fermentation broth was greater than 3.0 and less than 6.0. In addition, the viscosity analysis of the concentrated slurry confirmed that the viscosity was significantly lower when the pH of the fermentation broth was greater than 3.0 and less than 6.0. A lower viscosity of the concentrated slurry indicates that the valine crystals comprised in the concentrated slurry are large in size and that few fine crystals have formed. In contrast, in the fermentation broths with pH 7.0, pH 6.0, and pH 3.0, which had high viscosity of the concentrated slurry, it is analyzed that numerous fine valine crystals were formed, resulting in the high slurry viscosity.

Accordingly, it was found that the pH of the fermentation broth affects the formation of valine crystals. Specifically, it was found that valine crystals with a large size are formed within a certain pH range, resulting in low viscosity of the concentrated slurry and low moisture content of the separated crystals.

### Experimental Example 3: Comparison of moisture content in valine crystals according to the type of additive

To confirm the effect of the presence and/or type of additive during the process of preparing valine crystals from a culture broth comprising valine, culture broths were prepared by adding 2 wt% (based on the weight of L-valine) of polyvinyl alcohol (PVA), polyvinylpyrrolidone (PVP), or monosodium glutamate (MSG) to the fermentation broth prepared in Experimental Example 1, in which the pH had been adjusted to 3.8 by adding sulfuric acid. These culture broths were concentrated to a concentration of 200 g/L using a vacuum concentrator at a vacuum degree of 120 torr and at a rate of 30 g/L/hr. Subsequently, solid-liquid separation was performed at 1800 rpm for 10 minutes using a basket centrifuge to obtain L-valine crystals from the concentrated culture broths obtained above, and the moisture content of the separated L-valine crystals was determined by LOD analysis and compared (Table 3).

**[Table 3]**

| Additive | Moisture content in valine crystals (wt%) |
|---|---|
| No additive (control group) | 58.21 |
| PVA | 40.12 |
| PVP | 57.89 |
| MSG | 59.34 |

As shown in Table 3, the use of other additives did not result in a significant change in the moisture content in the crystals, whereas the crystals obtained from the process using PVA exhibited a moisture content reduced by 30% or more compared with the control group using no additive. This indicates that adding PVA to the culture broth prior to concentration is effective in reducing the moisture content in the final valine crystals obtained.

### Experimental Example 4: Comparison of moisture content in valine crystals according to the amount of PVA used

Using PVA, which showed the most outstanding effect in Experimental Example 3, the effect according to the amount of additive used was examined by varying the input amount. Specifically, culture broths prepared with different input amounts of 0.5 wt%, 1.5 wt%, 2.0 wt%, and 2.5 wt% (based on the weight of L-valine) were concentrated and separated as in Experimental Example 3 to obtain crystals, and the moisture content of the separated L-valine crystals was determined by LOD analysis (Table 4).

**[Table 4]**

| | | | | | |
|---|---|---|---|---|---|
| Amount of PVA added (wt%) | 0 | 0.5 | 1.5 | 2.0 | 2.5 |
| Moisture content in crystals (wt%) | 58.21 | 53.07 | 47.23 | 38.02 | 48.23 |

All samples within the tested range of PVA input exhibited reduced moisture content in the crystals compared with the control sample with no PVA addition (0 wt%), and a reduction rate of about 9.1% to 34.7% was observed.

Furthermore, the morphology of the crystals generated in the sample treated with 2 wt% PVA and the control with no PVA addition was observed using SEM (×30, JEOL, JCM-6000PLUS). As a result, while the control group with no PVA addition produced plate-shaped fine crystals, the group treated with 2 wt% PVA formed particles of a larger size that were closer to a spherical shape (FIG. 1).

### Experimental Example 5: Preparation of valine granules from the separated crystals

Valine crystals were produced in the same manner as in Experimental Example 4, and the separated valine crystals were mixed with pre-dried valine seeds (having a valine content of 75%) to prepare crystals each having moisture contents of 15% to 47%, after which valine mixed granule crystals were produced using a mixer granulator.

It was confirmed that mixed granules can be produced from all crystals having moisture contents of 15% to 47% using a mixer granulator.

**[Table 5]**

| | | | | | | |
|---|---|---|---|---|---|---|
| Moisture content (%) | 15.0 | 24.0 | 30.0 | 35.0 | 47.0 | |
| Equipment | Type | CVG Mixer | | | | |
| | rpm | 2000 | | | | |
| Presence of mixed granules | | ○ | ○ | ○ | ○ | ○ |

As set forth above, those skilled in the art will be able to understand that the present disclosure may be embodied in other specific forms without departing from the technical spirit or essential characteristics thereof. In this regard, the embodiments disclosed herein are only for illustrative purposes and should not be construed as limiting the scope of the present disclosure. The scope of the present disclosure should be construed as including the meaning and scope of the appended claims rather than the detailed description, and all changes or variations derived from the equivalent concepts fall within the scope of the present disclosure.

## Claims

1. A method for preparing valine crystals, comprising:
a step of preparing a fermentation broth comprising valine;
a step of adjusting the pH of the fermentation broth;
a step of adding polyvinyl alcohol (PVA) to the fermentation broth; and
a step of separating valine crystals comprising moisture from the fermentation broth.

2. The method according to claim 1,
wherein, in the step of adjusting the pH of the fermentation broth, the pH of the fermentation broth is adjusted to a pH greater than 3 and less than 6.

3. The method according to claim 1,
wherein the PVA is added in an amount of 0.1 wt% to 5 wt% based on the total weight of the fermentation broth.

4. The method according to claim 1,
wherein the method further comprises a step of concentrating the fermentation broth after the step of adding PVA to the fermentation broth.

5. The method according to claim 4,
wherein the concentration is performed by removing moisture in the fermentation broth at a rate of 30 g/L/hr to 150 g/L/hr.

6. The method according to claim 1,
wherein the valine crystals have a viscosity greater than 0 cP and less than 80 cP.

7. The method according to claim 6,
wherein the valine crystals comprise 10 wt% to 55 wt% of moisture.

8. The method according to claim 1,
wherein the valine crystals have a spherical particle form.

9. A method for preparing valine granules, comprising:
a step of preparing a fermentation broth comprising valine;
a step of adjusting the pH of the fermentation broth;
a step of adding polyvinyl alcohol (PVA) to the fermentation broth;
a step of separating valine crystals comprising moisture from the fermentation broth;
a step of preparing mixed granules by mixing the valine crystals with seeds; and
a step of drying the mixed valine granules.

10. The method according to claim 9,
wherein the method further comprises a step of concentrating the fermentation broth after the step of adding PVA to the fermentation broth.
